# EUROPÄISCHE PATENTSCHRIFT

(11) **EP 1 731 115 B1**
(45) Veröffentlichungstag und Bekanntmachung des Hinweises auf die Patenterteilung: **26.03.2008**
(21) Anmeldenummer: 06007925.8
(22) Anmeldetag: 15.04.2006
(51) Int. Cl.: A61F 2/42

(54) **Zementfreie Tibiakomponente für eine Sprunggelenkprothese sowie mit einer solchen gebildete Sprunggelenkprothese**
Cement-free tibial component for an ankle replacement prosthesis and an ankle prosthesis comprising such a component
Elément tibial non cimentée pour prothèse de l'articulation de cheville et une prothèse de l'articulation de cheville comprenant cet élément

(30) Priorität: 08.06.2005 DE 102005026566
(43) Veröffentlichungstag der Anmeldung: 13.12.2006
(73) Patentinhaber: implantcast GmbH, 21614 Buxtehude (DE)
(72) Erfinder: Fink, Bernd Prof. Dr., 71665 Vaihingen/Enz (DE); Schill, Stephan Dr., 83043 Bad Aiblingen (DE); Schenk, Katja Dr., 39110 Magdeburg (DE); Rehart, Stefan Priv.-Doz. Dr., 65719 Hofheim a. Ts. (DE)
(74) Vertreter: von Eichel-Streiber, Caspar

(56) Entgegenhaltungen:
- WO-A-20/05030098
- FR-A- 2 676 917
- US-A1- 2004 002 768

## Beschreibung

Die Erfindung betrifft eine zementfreie Tibiakomponente für eine Sprunggelenkprothese. Sie betrifft ferner eine mit einer solchen Tibiakomponente gebildete Sprunggelenkprothese.

Einesteils wegen ständig steigender Lebenserwartung und altersbedingter Ausfälle, anderenteils wegen krankhafter Veränderungen oder Verletzungen, beispielsweise Sportverletzungen, ist es immer häufiger erforderlich, bei Patienten ein nicht mehr gebrauchstaugliches, natürliches Sprunggelenk durch eine Prothese zu ersetzen. Derartige Prothesen sind hinlänglich bekannt, ein Beispiel für eine solche Prothese ist in der europäischen Patentschrift EP 0 864 304 B1 offenbart.

Die vorliegende Erfindung betrifft solche Sprunggelenkprothesen, die zwischen dem Schienbein (Tibia) des Unterschenkels und dem Sprungbein (oberster Fußwurzelknochen, Talus) des Fußes eingesetzt werden, insbesondere betrifft die Erfindung die Tibiakomponente einer solchen Sprunggelenkprothese, genauer eine zementfreie Tibiakomponente für eine solche Sprunggelenkprothese.

Während einer Operation zum Implantieren einer Sprunggelenkprothese werden zunächst die defekten Gelenkteile des Sprunggelenks von Tibia und Talus entfernt. Anschließend werden die jeweiligen Komponenten der Sprunggelenkprothese mit den zugehörigen Knochen verbunden. Im Falle einer zementfreien Tibiakomponente erfolgt diese Verbindung ohne Einsatz von die Komponente mit dem Knochen verbindendem Zement oder Kleber, die Prothesenkomponente hält ausschließlich durch "Verkeilung" in dem Knochen.

Eine solche Prothese ist aus der veröffentlichten Patentanmeldung US 2004/0002768 A1 bekannt.

Aufgabe der vorliegenden Erfindung ist es, eine zementfreie Tibiakomponente für eine Sprunggelenkprothese anzugeben mit einer Befestigungsstruktur, die einen sicheren Halt im Knochenmaterial des Schienbeins sowie eine hohe Primärstabilität bei möglichst sparsamer Knochenresektion ermöglicht.

Diese Aufgabe wird gelöst durch eine zementfreie Tibiakomponente für eine Sprunggelenkprothese mit den Merkmalen des Anspruches 1.

Die erfindungsgemäß auf der Basis der Tibiakomponente angeordneten Befestigungsfinnen weisen zueinander bzw. damit auch zu der Basis jeweils einen solchen Winkel auf, der eine hohe Primärstabilität gewährleistet und zudem eine gute Verankerung im Knochenmaterial der Tibia gewährleistet.

Grundsätzlich genügt ein Paar einander gegenüberliegender Befestigungsfinnen. Allerdings ist es ebenso gut möglich, zwei oder mehr Paare von Befestigungsfinnen fluchtend hintereinander anzubringen, so dass alle Befestigungsfinnen paarweise einander gegenüberliegen und entsprechend den Merkmalen des Anspruches 1 zueinander geneigt angeordnet sind. Gleichermaßen ist es ebenfalls denkbar, die paarweise einander gegenüberliegenden Befestigungsfinnen versetzt anzuordnen.

Für ein vereinfachtes Verbinden der erfindungsgemäßen Tibiakomponente mit dem Schienbeinknochen sind die Befestigungsfinnen vorzugsweise trapezförmig gebildet und liegen mit ihrer breiteren Seite an der Basis an (Anspruch 2).

Gemäß einer vorteilhaften Weiterbildung, wie sie in Anspruch 3 angegeben ist, weisen die dort angegebenen Befestigungsfinnen an der dort beschriebenen Schmalkante seitliche Fasen auf. Diese Fasen vereinfachen zum einen das Einschlagen der Tibiakomponente in den Schienbeinknochen (sie dienen als Einführhilfe), sie beeinflussen zum anderen zusammen mit der Stellung der Befestigungsfinnen die Einschlagrichtung so, dass die Tibiakomponente optimal an die Resektionsfläche des Schienbeinknochens herangezogen wird.

Hinsichtlich einer vollständigen Sprunggelenkprothese wird die eingangs genannte Aufgabe durch eine Sprunggelenkprothese mit den Merkmalen des Anspruches 7 gelöst. Neben der erfindungsgemäßen, zementfreien Tibiakomponente weist diese Sprunggelenkprothese noch eine Taluskomponente sowie ein zwischen der Tibiakomponente und der Taluskomponente angeordnetes Lagerteil auf.

Sowohl die Tibiakomponente als auch die Taluskomponente sind vorzugsweise aus einer CoCrMo-Gusslegierung gefertigt (Ansprüche 4 bzw. 9).

Die genannten Komponenten können für eine bessere Osteointegration zumindest teilweise mit Reintitan und/oder Hydroxy-apatite beschichtet sein, alternativ für bessere Verschleißeigenschaften auch mit einer keramischen Titannitritschicht (Anspruche 5, 6 bzw. 10).

Das Lagerteil der erfindungsgemäßen Sprunggelenkprothese besteht vorzugsweise aus Polyäthylen (Anspruch 8).

Weitere Vorteile und Merkmale der Erfindung ergeben sich aus der nachfolgenden Beschreibung eines Ausführungsbeispiels anhand der beigefügten Figuren. Dabei zeigen:
- Fig. 1: eine erfindungsgemäße, zementfreie Tibiakomponente für eine Sprunggelenkprothese in einer Ansicht von vorn;
- Fig. 2: die in Fig. 1 gezeigte Tibiakomponente in einer Ansicht von oben;
- Fig. 3: die in Fig. 1 gezeigte Tibiakomponente in einer Ansicht von der Seite;
- Fig. 4: die in Fig. 1 gezeigte Tibiakomponente in einer dreidimensionalen Ansicht schräg von vorn;
- Fig. 5: eine unter Verwendung der in den Figuren 1 bis 4 gezeigten Tibiakomponente gebildete Sprunggelenkprothese;
- Fig. 6: eine Ansicht der in Fig. 5 gezeigten Sprunggelenkprothese von vorn (von links in Fig. 5);
- Fig. 7: eine entlang der Linie VII - VII in Fig. 6 genommene Schnittansicht der Sprunggelenkprothese; und
- Fig. 8: eine dreidimensionale Ansicht der Sprunggelenkprothese aus Fig. 5 schräg von vorn.

In den Figuren sind gleiche Elemente mit gleichen Bezugszeichen versehen.

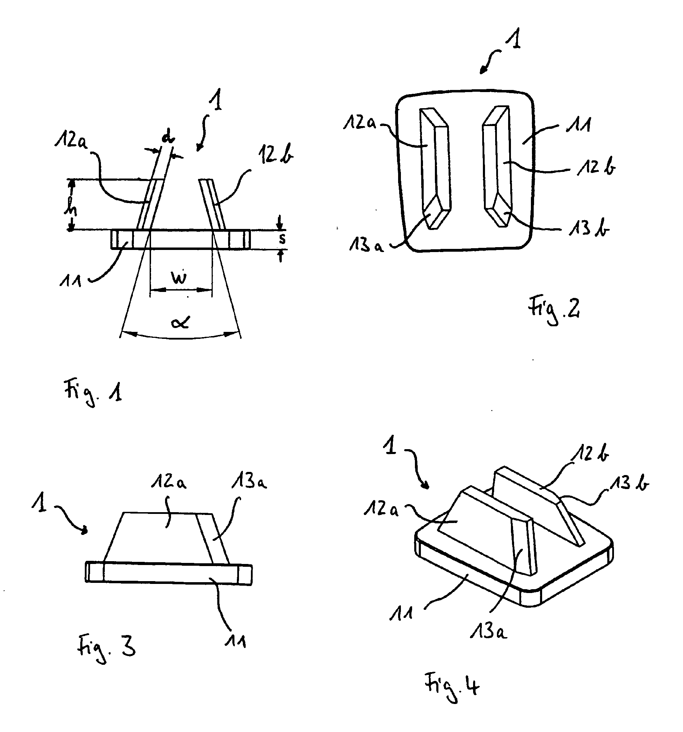

In den Figuren 1 bis 4 ist zunächst in verschiedenen Ansichten ein Ausführungsbeispiel für eine erfindungsgemäße, zementfreie Tibiakomponente 1 für eine Sprunggelenkprothese dargestellt. Diese Komponente enthält eine ebene, plattenförmige Basis 11 sowie ein Paar aus zwei schräg zu der Basis verlaufenden Befestigungsfinnen 12a und 12b zur Verankerung der Tibiakomponente 1 im Knochenmaterial des Schienbeins. Wie in Kombination der Ansichten Fig. 2 und Fig. 1 zu erkennen ist, sind die Befestigungsfinnen 12a und 12b einander gegenüberliegend paarweise angeordnet und zueinander geneigt. Dabei liegen die Befestigungsfinnen 12a, 12b, wie besonders gut in Fig. 1 zu erkennen ist, auf den Schenkeln eines gedachten, gleichschenkligen Dreiecks, dessen Grundseite durch die Oberfläche der Basis 11 gebildet wird. Der zwischen den Schenkeln dieses gleichschenkligen Dreiecks eingeschlossene Winkel ist mit α bezeichnet und beträgt in diesem Ausführungsbeispiel exakt 30°.

In Fig. 3 am besten zu erkennen ist, dass die Befestigungsfinnen 12a, 12b trapezförmig gebildet sind, wobei deren längere Kante an der Basis 11 anliegt.

In Fig. 2 ist zu erkennen, dass die Basis 11 der Tibiakomponente 1 in der dort unten dargestellten Querkante etwas schmaler ausgebildet ist als in der oben dargestellten Querkante. In diesem Ausführungsbeispiel ist die Basis 11 an der Unterkante 24,7 mm, an der Oberkante 27 mm breit. Die Länge der Basis 11 beträgt 32 mm. Der Abstand der Befestigungsfinnen 12a, 12b von der Fig. 2 oben dargestellten, längeren Querkante der Basis 11 beträgt 3,5 mm.

Die Basis selbst weist eine Stärke s von 4 mm auf. Der Abstand der einander gegenüberliegenden Befestigungsfinnen 12a, 12b ist in Fig. 1 mit w bezeichnet und beträgt in diesem Ausführungsbeispiel 12 mm. Die in Fig. 1 mit h bezeichnete Höhe der Befestigungsfinnen 12a bzw. 12b über der Basis 11 beträgt in diesem Ausführungsbeispiel 10 mm. Die Befestigungsfinnen 12a und 12b weisen eine Dicke d von 2,5 mm auf.

In Fig. 2 und 4 ist am besten zu erkennen, dass die Befestigungsfinnen 12a und 12b ausgehend von ihren vorderen Schmalkanten an der jeweiligen Außenseite mit Fasen 13a und 13b versehen sind. Diese stellen eine Einführhilfe dar und beeinflussen die Einschlagrichtung beim Einschlagen der Tibiakomponente 1 derart, dass diese an die Resektionsfläche des Schienbeines herangezogen wird.

Durch die spezielle Anordnung der erfindungsgemäßen Befestigungsfinnen 12a, 12b sind ein besonders guter Halt der zementfreien Tibiakomponente 1 im Knochen des Schienbeins sowie eine hohe Primärstabilität gewährleistet.

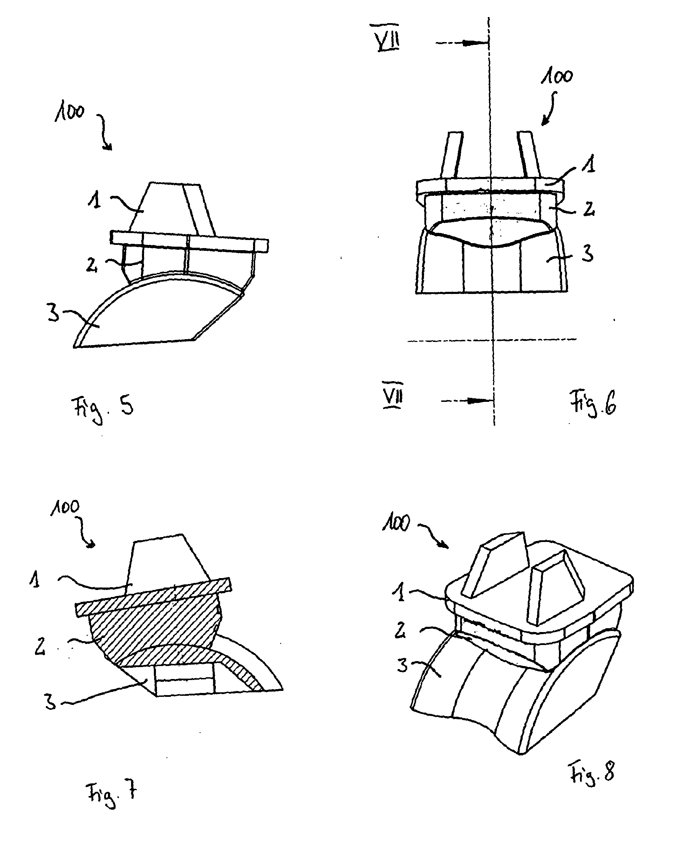

In den Figuren 5 bis 8 ist in verschiedenen Ansichten eine Sprunggelenkprothese 100 dargestellt, welche eine in den Figuren 1 bis 4 gezeigte Tibiakomponente 1 enthält. Neben der Tibiakomponente 1 enthält die Sprunggelenkprothese noch eine Taluskomponente 3 sowie ein zwischen der Tibiakomponente 1 und der Taluskomponente 3 angeordnetes Lagerteil 2.

Sowohl die Tibiakomponente 1 als auch die Taluskomponente 3 der Sprunggelenkprothese 100 sind aus einer CoCrMo-Gusslegierung hergestellt. Das Lagerteil 2 besteht aus Polyethylen.

Zur Verbesserung der Osteointegration können sowohl die Tibiakomponente 1 als auch die Taluskomponente 3 mit einer Beschichtung aus Reintitan und/oder Hydroxyapatite versehen sein, wobei diese Beschichtung teilweise oder vollständig auf der entsprechenden Komponente aufgetragen sein kann. Alternativ können die genannten Teile auch teilweise oder vollständig mit einer keramischen Titannitritschicht beschichtet sein, um verbesserte Verschleißeigenschaften zu erhalten.

Das gezeigte Ausführungsbeispiel dient lediglich der Erläuterung und ist nicht beschränkend.

### Bezugszeichenliste

- 1: Tibiakomponente
- 11: Basis
- 12 a,b: Befestigungsfinne
- 13 a,b: Fase

- 2: Lagerteil
- 3: Taluskomponente

- 100: Sprunggelenkprothese

- α: Winkel

## Patentansprüche

1. Zementfreie Tibiakomponente für eine Sprunggelenkprothese mit einer Basis (11) sowie mit wenigstens einem Paar von einander gegenüberliegenden Befestigungsfinnen, **dadurch gekennzeichnet, dass** die Befestigungsfinnen als schräg zu der Basis (11) verlaufende, im wesentlichen plattenförmige, aufeinander zu geneigte Befestigungsfinnen (12a, 12b) ausgestaltet sind, und wobei die Befestigungsfinnen (12a, 12b) in einer Ansicht senkrecht zu ihrer Plattenebene entlang solcher Linien verlaufen, die in Verlängerung Schenkel eines gleichschenkeligen Dreiecks bilden, dessen Grundlinie die Basis (11) bildet, wobei der von den Schenkeln eingeschlossene, der Basis gegenüberliegende Winkel (α) in einem Bereich von 25° bis 35° liegt, vorzugsweise 30° beträgt.

2. Zementfreie Tibiakomponente nach Anspruch 1, **dadurch gekennzeichnet,** die Befestigungsfinnen (12a, 12b) in einer Seitenansicht trapezförmig gebildet sind und mit ihrer breiteren Seite an der Basis (11) anliegen.

3. Zementfreie Tibiakomponente nach einem der vorhergehenden Ansprüche, **dadurch gekennzeichnet, dass** die Befestigungsfinnen (12a, 12b) an einer in Einbaulage am vorderen Teil des Schienbeins (Tibia) gelegenen Schmalkante auf den voneinander abgewandten Außenseiten angefast (13a, 13b) sind.

4. Zementfreie Tibiakomponenten nach einem der vorhergehenden Ansprüche, **dadurch gekennzeichnet, dass** sie aus einer CoCrMo-Gußlegierung gefertigt ist.

5. Zementfreie Tibiakomponente nach einem der vorhergehenden Ansprüche, **dadurch gekennzeichnet, dass** sie zumindest teilweise mit Reintitan und/oder Hydroxyapatite beschichtet ist.

6. Zementfreie Tibiakomponente nach einem der Ansprüche 1 bis 3, **dadurch gekennzeichnet, dass** sie zumindest teilweise mit einer Schicht aus keramischem Titannitrit beschichtet ist.

7. Sprunggelenkprothese bestehend aus einer zementfreien Tibiakomponente (1) nach einem der vorhergehenden Ansprüche, einer Taluskomponente (3) sowie einem zwischen der Tibiakomponente (1) und der Taluskomponente (2) zwischengeordneten Lagerteil (2).

8. Sprunggelenkprothese nach Anspruch 7, **dadurch gekennzeichnet, dass** das Lagerteil (2) aus Polyäthylen besteht.

9. Sprunggelenkprothese nach einem der Ansprüche 7 oder 8, **dadurch gekennzeichnet, dass** die Taluskomponente (3) aus einer CoCrMo-Gußlegierung gefertigt ist.

10. Sprunggelenkprothese nach einem der Ansprüche 7 bis 9, **dadurch gekennzeichnet, dass** die Taluskomponente (3) zumindest teilweise mit Reintitan und/oder Hydroxyapatite beschichtet ist oder alternativ zu einem oder beiden der erstgenannten Materialien zumindest teilweise mit einer Schicht aus keramischem Titannitrit beschichtet ist.

## Claims

1. Cement-free tibial component for an ankle prosthesis with a base (11) and at least one pair of mutually facing fastening fins, **characterized in that** the fastening fins are designed as substantially plate-like fastening fins (12a, 12b) inclined towards one another and sloping with respect to the base (11), wherein a view perpendicular to their plate plane, said fastening fins (12a, 12b) run along lines such that in extension they form sides of an isosceles triangle, whose base line forms the base (11), the angle (α) facing the base and enclosed by the sides being in a range 25 to 35°, preferably 30°.

2. Cement-free tibial component according to claim 1, **characterized in that**, in a side view, the fastening fins (12a, 12b) have a trapezoidal formation and engage on the base (11) with their wider side.

3. Cement-free tibial component according to one of the preceding claims, **characterized in that** the fastening fins (12a, 12b) on a narrow edge which, in the assembly position, is located on the front part of the shinbone (tibia) are chamfered (13a, 13b) on the mutually remote outsides.

4. Cement-free tibial component according to one of the preceding claims, **characterized in that** it is made from a CoCrMo cast alloy.

5. Cement-free tibial component according to one of the preceding claims, **characterized in that** it is at least partly coated with pure titanium and/or hydroxyapatite.

6. Cement-free tibial component according to one of the claims 1 to 3, **characterized in that** it is at least partly coated with a layer of ceramic titanium nitrite.

7. Ankle prosthesis comprising a cement-free tibial component (1) according to one of the preceding claims, a talus component (3) and a bearing part (2) placed between the tibial component (1) and talus component (3).

8. Ankle prosthesis according to claim 7, **characterized in that** the bearing part (2) is made from polyethylene.

9. Ankle prosthesis according to one of the claims 7 or 8, **characterized in that** the talus component (3) is made from a CoCrMo cast alloy.

10. Ankle prosthesis according to one of the claims 7 to 9, **characterized in that** the talus component (3) is at least partly coated with pure titanium and/or hydroxyapatite or alternatively as an alternative to one or both of the first-mentioned materials, is at least partly coated with a layer of ceramic titanium nitrite.

## Revendications

1. Elément tibial non cimenté pour une prothèse d'articulation de cheville avec une base (11) et avec au moins une paire de pannes de fixation opposées l'une à l'autre, **caractérisé en ce que** les pannes de fixation sont conçues comme des pannes de fixation (12a, 12b) sensiblement en forme de plaques, inclinées l'une vers l'autre, s'étendant en biais par rapport à la base (11) et dans lequel les pannes de fixation (12a, 12b) s'étendent, selon une vue perpendiculaire à leur plan de plaque, le long de lignes qui forment dans le prolongement des côtés d'un triangle isocèle dont la ligne de base forme la base (11), dans lequel l'angle (α) opposé à la base, inclus par les côtés, se trouve dans une région comprise entre 25° et 35° et est, de préférence, de 30°.

2. Elément tibial non cimenté selon la revendication 1, **caractérisé en ce que** les pannes de fixation (12a, 12b) ont une forme trapézoïdale selon une vue de côté et s'appuient sur la base (11) avec leurs côtés les plus larges.

3. Elément tibial non cimenté selon l'une quelconque des revendications précédentes, **caractérisé en ce que** les pannes de fixation (12a, 12b) sont biseautées (13a, 13b) sur une arête étroite placée sur la partie avant du tibia en position de montage sur les côtés externes opposés l'un à l'autre.

4. Elément tibial non cimenté selon l'une quelconque des revendications précédentes, **caractérisé en ce qu'**il est fait en un alliage de fonderie CoCrMo.

5. Elément tibial non cimenté selon l'une quelconque des revendications précédentes, **caractérisé en ce qu'**il est revêtu au moins partiellement avec du titane pur et/ou une hydroxyapatite.

6. Elément tibial non cimenté selon l'une quelconque des revendications 1 à 3, **caractérisé en ce qu'**il est revêtu au moins partiellement avec une couche de nitrite de titane céramique.

7. Prothèse d'articulation de cheville constituée d'un élément tibial non cimenté (1) selon l'une quelconque des revendications précédentes, d'un élément d'astragale (3) ainsi que d'une pièce d'appui (2) placée entre l'élément de tibia (1) et l'élément d'astragale (3).

8. Prothèse d'articulation de cheville selon la revendication 7, **caractérisée en ce que** la pièce d'appui (2) est faite en polyéthylène.

9. Prothèse d'articulation de cheville selon l'une quelconque des revendications 7 ou 8, **caractérisée en ce que** l'élément d'astragale (3) est fait en un alliage de fonderie CoCrMo.

10. Prothèse d'articulation de cheville selon l'une quelconque des revendications 7 à 9, **caractérisée en ce que** l'élément d'astragale (3) est revêtu au moins partiellement avec du titane pur et/ou une hydroxyapatite ou, en variante à l'un de matériaux mentionnés en premier ou aux deux matériaux, **en ce qu'**il est revêtu au moins partiellement avec une couche de nitrite de titane céramique.
